Europäisches Patentamt

(19) European Patent Office

Office européen des brevets

(11) Publication number : **0 380 068 B1**

## (12) EUROPEAN PATENT SPECIFICATION

(45) Date of publication of patent specification :
**30.09.92 Bulletin 92/40**

(51) Int. Cl.⁵ : **C12N 15/85, C12N 15/13, C12P 21/08, C12N 5/12**

(21) Application number : **90101351.6**

(22) Date of filing : **24.01.90**

(54) **An expression system for production of chimeric monoclonal antibodies.**

(30) Priority : **24.01.89 US 301216**
**04.12.89 US 441702**

(43) Date of publication of application :
**01.08.90 Bulletin 90/31**

(45) Publication of the grant of the patent :
**30.09.92 Bulletin 92/40**

(84) Designated Contracting States :
**AT BE CH DE DK ES FR GB GR IT LI LU NL SE**

(56) References cited :
**EP-A- 0 256 654**
**EP-A- 0 338 745**
**WO-A-89/09622**

(56) References cited :
**PROTEIN ENGINEERING, vol. 1, no. 6, December 1987, pages 499-505, IRL Press Ltd, Eynsham, Oxford, GB; N. WHITTLE et al.:"Expression in COS cells of a mouse-human chimaeric B72.3 antibody"**

(73) Proprietor : **MILES INC.**
**One Mellon Center 500 Grant Str.**
**Pittsburgh, PA 15219-2502 (US)**

(72) Inventor : **Zerler, Bradley, Dr.**
**3222 Whitney Avenue**
**Hamden, CT 06514 (US)**

(74) Representative : **Dänner, Klaus, Dr. et al**
**c/o Bayer AG Konzernverwaltung RP Patente Konzern**
**W-5090 Leverkusen 1 Bayerwerk (DE)**

## Description

The present invention relates to an expression system which provides a method for the production of chimeric monoclonal antibodies. The expression system includes vectors that contain the entire genomic human constant region of a human immunoglobulin heavy and/or light chain and is adaptable for insertion of the entire variable region of a nonhuman antibody. The expression vector contains the entire genomic constant region of the human antibody, and the entire variable region of the nonhuman antibody lacking tissue specific promoter and/or enhancer regions normally associated with variable region genomic DNA. In addition, this expression system does not require creation of restriction enzyme recognition sites in the variable region of the cDNA by mutagenesis. Host cells containing the expression vector and producing immunoglobulin can include lymphocyte or nonlymphocyte cell lines. Monoclonal antibodies against interleukin-2 receptor and tumor necrosis factor are examples of chimeric monoclonals that can be produced by this system.

An antibody consists of heavy and light chain polypeptides which associate to produce a functional molecule. Both the heavy and light chains contain two regions designated the variable region and the constant region. The variable regions of the heavy and light chain bind to a specific antigen. The heavy and light chain constant regions are not involved in antigen binding, but the heavy chain constant region has a physiological role through specific effector functions. In producing a chimeric antibody, the heavy and light chain constant regions of a human antibody are coupled with the variable region of a nonhuman antibody to achieve the desired binding function of the nonhuman antibody while reducing the likelihood of an immunogenic response to the nonhuman constant region. Human effector functions are also preserved. Prior art methods have provided chimeric antibodies having a human constant region and a nonhuman variable region, but none of the prior art methods known to date either provide an expression vector that is adaptable for insertion of the variable region without mutagenesis of the variable region, or which lack a tissue specific promoter. Additionally, this vector allows for subcloning and expression of variable region genes which lack enhancer regions normally associated with genomic DNA.

WO 86/01533 (Celltech Limited) describes a process for the production of chimeric antibodies using recombinant techniques. They define the term "chimeric antibody" to describe a protein comprising at least the antigen binding protein of an immunoglobulin molecule (Ig) attached to at least part of another protein. In this example, the constant and variable region genes include tissue specific promoter and enhancer regions associated with genomic DNA. These tissue specific regions limit the use of the expression vector to lymphocytes as the host cell.

EPO 0 125 023 (Genentech) describes the use of recombinant DNA techniques in bacterial cells to produce Igs and proposed the construction of chimeric Igs. Celltech in WO 86/01533 believed that the Genentech application did not result in the secretion and assembly of the chains into the desired chimeric Igs. An explanation for the inability to secrete and assemble the chains into the desired chimeric Igs is the inability of a procaryotic cell to correctly process a mammalian secreted protein.

Liu et al, Proc. Natl. Acad. Sci. Vol. 84, pp 3439-3443 (May 1987), disclose a chimeric mouse-human antibody using cDNAs that were introduced into lymphoid cells by DNA transfection. The cDNAs were isolated and restriction enzyme recognition sites created in the cDNA sequences at the V/C junction by in vitro mutagenesis. This process requires that the nucleotide sequence be known for each variable region and that each variable region be altered by mutagenesis.

United States Patent U.S. 4,085,707 (Waldmann) describes a method for treating malignancy and autoimmune disorders and for preventing allograft rejection using conjugated or unconjugated monoclonal antibodies to interleukin-2 receptor. Heterologous murine monoclonal antibodies are prepared from the ascites fluid of BALB/C mice and purified by DEAE chromatography. The patent specifically describes the treatment of patients with HTLV-I associated Adult T-Cell Leukemia (ATL) using intravenously administered anti-Tac monoclonal antibody (Treatment A); using anti-Tac monoclonal coupled to ricin toxin A-chain or Pseudomonas toxin (Treatment B); and using anti-Tac monoclonal coupled to bismuth-212 or Yttrium-90 radionuclides (Treatment C).

Pastan et al, U.S. Patent 4,545,985 describes and claims immunotoxin conjugates of Pseudomonas exotoxin with epidermal growth factor, transferrin receptor monoclonal antibody and interleukin-2 receptor monoclonal antibody (anti-Tac), for use in chemotherapy. The preparation of murine monoclonal antibody to interleukin-2 receptor was prepared as described by Uchiyama et al in the J. of Immunol 126:11393 (1981).

Diamanstein and Osawa (Bayer), GB Patent Application 2188941 describes and claims compositions of at least two (2) monoclonal antibodies recognizing the interleukin-2 receptor which are capable of inhibiting interleukin-2 dependent lymphocyte proliferation for the treatment of hyperimmune syndrome diseases. The application describes the preparation and characterization of murine monoclonal antibodies from the two hybridoma clones AHT-54 and AHT-107 deposited with the NTCC, No. 86041801 and 86041802, respectively, which recognize different binding sites (epitopes) on the interleukin-2 receptor. The application also discloses that the combination of monoclonal antibodies AHT-54 and AHT-107 results in greater inhibition of interleukin-2 dependent lymphocyte proliferation. Although monoclonal antibodies to the interleukin-2 receptor having heavy and light chain human constant regions and murine variable regions are proposed, no experiments were conducted to generate such antibodies.

European Patent Application No. 235805 (Janossy of the Royal Free Hospital in London) describes and claims compositions of a plurality of monoclonals to T-cell surface antigens for immunosuppressive therapy to patients having undergone kidney transplantation. An alleged advantage of the invention is the use of monoclonal antibodies recognizing different epitopes on the cell surface antigen. Although chimeric monoclonal antibodies to T-cell surface antigens are disclosed, again there are no experiments disclosed which specifically demonstrate their efficacy. The description of chimeric monoclonal antibodies is limited to a hypothetical monoclonal antibody having a human constant region and a murine variable region.

European Patent Application 217992 (Strom of Beth Israel Hospital) discloses and claims, interalia, monoclonal antibodies to the interleukin-2 receptor for inhibiting binding of interleukin-2 to T-lymphocytes for treating allograft rejection. Murine monoclonal antibody M7/20, specific for the IL-2 receptor was administered to mice after heterotropic heart allographs were performed.

Although the strategy of using monoclonal antibodies to bind interleukin-2 receptor to suppress T cell proliferation has been described in detail, this is not the case for chimeric monoclonal antibodies.

Activated T-lymphocytes have a major role in many undesirable immune responses. Anti-IL-2 receptor antibody therapy has advantages over other standard therapies such as drugs or radiation, because the IL-2 receptor antibody can selectively bind to and facilitate the removal of only those T-cells actively involved in the immune response while not having an effect on resting T-cells or other tissue. Diamanstein and Osawa, supra, have achieved the above desired result using murine monoclonal antibodies. Murine monoclonal antibodies have been utilized because of the difficulties of producing human monoclonals of appropriate specificity. Since the mouse antibodies are viewed as foreign proteins by the human immune system, the mouse antibodies often evoke counteracting antibodies that destroy their effectiveness and may also cause allergic side effects. Another important advantage of chimeric monoclonals is the increased serum half-life. A murine monoclonal IgG-1 antibody has a serum half-life of 15-16 hours in humans whereas a human IgG-1 monoclonal antibody has a serum half-life of 21-23 days.

To decrease the possibility of a counteracting immune response against the anti-IL-2 receptor antibodies the IL-2 receptor antibodies will be humanized by making amouse:human chimeric as disclosed by the expression system of the present invention. By substituting the human light chain and heavy chain constant regions for the respective mouse constant regions the antigenic problems associated with using a mouse monoclonal antibody in humans will be greatly reduced.

U.S. Patent 4,603,106, Cerami et al (Rockefeller Univ) discloses and claims an antibody to a defined mediator. Although the term tumor necrosis factor (TNF) is not used in the patent, the characterized mediator is believed to be TNF. The patent points out that antibodies to the mediator can be produced by known techniques, including the use of hybridomas utilizing, for example, fused mouse spleen cells and myeloma cells. There is no reference to chimeric monoclonal antibodies in this patent.

SUMMARY OF THE INVENTION

An expression system is provided for producing chimeric monoclonal antibodies. The expression system includes expression vectors which comprise the entire constant region of a human immunoglobulin heavy and/or light chain, said expression vector is adaptable for insertion, without mutagenesis, of a nonhuman antibody variable region such that the resulting expression vector includes the entire human genomic constant region and the entire nonhuman variable region lacking the tissue specific promoter and/or enhancer regions normally associated with genomic immunoglobulin DNA.

The expression vector further includes a promoter upstream of said human constant region, the promoter linked to said constant region by a polylinker containing at least one restriction site, unique to said vector and to said variable region. An example of such a polylinker is m13MP7 polylinker which provides a plurality of restriction sites. It is recognized that other polylinkers, containing multiple cloning sites (mcs) could provide the same function.

The expression system is adaptable for insertion of the variable region, said variable region to be inserted includes a synthetic oligonucleotide linker such that when said variable region and said linker are inserted between the promoter and the constant region the resulting vector includes the entire constant region fused in-frame with the entire variable region lacking tissue specific regions normally associated with genomic DNA.

The expression system further includes a restriction site, introduced into the human constant region, said restriction site unique to said vector including said human constant region and said variable region. An example of such a restriction site is Cla I. It is recognized that other restriction sites could provide the same function.

The assembled vector with human constant region containing a unique restriction site, and promoter separated by a polylinker such as, m13 polylinker containing mcs, provides a generic vector for the production of chimeric monoclonal antibodies. The vector including the above described cassette allows cloned nonhuman variable regions, lacking tissue specific regulatory elements normally associated with genomic DNA, to be inserted between the promoter and the constant region without the insertion of a restriction enzyme recognition site in the variable region. The expression system provides a method for cloning and inserting any variable region lacking the tissue specific promoters and/or enhancers normally associated with genomic DNA without insertion of a restriction enzyme recognition site in the variable region. Thus, this generic vector has the advantage of being suitable for transfection in lymphocyte and nonlymphocyte host cells for production of immunoglobulins. The system allows for the insertion of cloned variable regions using conventional recombinant techniques for cloning. It also provides an expression vector for the insertion of nucleotide sequences isolated using polymerase chain reaction technology.

An object of the present invention is to provide a generic expression system that allows for insertion of a nonhuman variable region without mutagenesis of the variable region.

A further object of the present invention is to provide an expression vector that can be introduced into lymphocyte and nonlymphocyte cells by DNA transfection.

## BRIEF DESCRITPION OF THE INVENTION

Figure 1 is the nucleotide sequence and amino acid sequence of the variable region of AHT107 heavy chain.

Figure 2 is the nucleotide sequence and amino acid sequence of the variable region of AHT54 heavy chain.

Figure 3 is the nucleotide sequence and amino acid sequence of the variable region of AHT107 light chain.

Figure 4 is the nucleotide sequence and amino acid sequence of the variable region of AHT54 light chain.

Figure 5 is a diagrammatic representation of chimaeric antibody expression vectors.

Figure 6 is a diagrammatic representation of an expression vector (designated pMTI-3023) containing the TAT gene under transcriptional regulation of the metallothionein promoter, and the GPT drug resistance marker under transcriptional regulation of the SV 40 promoter.

## DETAILED DESCRIPTION OF PREFERRED EMBODIMENTS

Murine hybridomas designated AHT54 and AHT107 as described by Diamanstein and Osawa, supra, were used to obtain the nonhuman variable regions.

Non-limiting examples of heterologous promoters for use in the present invention include the following:

(1) mouse and human metallothionein;

(2) long terminal repeats (LTR) of Rous sarcoma, Moloney leukemia, Moloney sarcoma and other retroviruses;

(3) early and late promoters of SV40 and polyoma viruses;

(4) immediate early promoter of human cytomegalovirus;

(5) major late promoter of adenovirus;

(6) combinations of enhancer elements and core promoter elements from promoters (1) to (5) above;

(7) combinations of immunoglobulins, gene enhancers and control elements with promoter elements of the above described promoters.

Non-limiting examples of cell lines for use in the present invention include the following permanent cell lines which are derived from human, rodent, simian and other mammalian species;

Human: HeLa, 293 myleomas

Rodent: Mouse: NIH 3T3, C127,MOP, myelomas.

Hamster: CHO, BHK.

Rat: Myelomas, Rat 2

Simian: CV-1, COS, VERO

## mRNA Isolation

Suspensions containing $10^9$ cells were pelleted by centrifugation at 1500 rpm for 15 min. To the cell pellet containing $10^9$ AHT107 cells add 30 ml LST buffer (a list of all buffer solutions is listed below) and 2 ml Vanadyl Ribonucleoside Complex (Bethesda Research Laboratories, Gaithersburg, MD) and gently resuspend cells; keeping the cells on ice. Transfer the resuspended cells to a 50 ml tube. Add 12 ml 4X TNLB buffer inverting gently 10 times. Centrifuge the resuspended cells at 2500 RPM for 5 min. Transfer supernatant to a 250 ml centrifuge bottle with cap. Add 4 ml 10% Sodium Dodecyl Sulfate (SDS) to the supernatant and bring volume up to 80 ml with ACE buffer. Add 80 ml ACE/Phenol and shake vigorously for 5 min at room temperature. Centrifuge at 5000 RPM for 10 min. Remove the aqueous phase and transfer to fresh 250 ml bottle. Precipitate the RNA with 2 volumes of -20°C absolute ethanol and add 1/10 the aqueous volume, 1 M NaCl. Precipitate the RNA overnight at -20°C. Pellet the RNA by centrifugation at 7000 RPM for 40 min at 4°C. Wash the pellet with 70% ethanol and centrifuge 7000 RPM for 15 min at 4°C. Dry the pellet (lyophilize). Resuspend the pellet containing total RNA in $dH_2O$.

Poly(A)$^+$ mRNA is isolated using oligo(dT)-cellulose chromatography according to the protocol described in Maniatis et al.

## cDNA Synthesis and Construction of Lambda GT10 Library

Double stranded cDNA was synthesized using a Bethesda Research Laboratories (BRL, Gaithersburg, Maryland) cDNA Synthesis System (catalogue number 8267SA). Size select double stranded cDNA by fractionation through a 6% polyacrylamide gel. Excise a gel fragment containing DNA 500 bp and larger. Electroelute DNA from gel for 2 h at 140 Volts in 0.1X TBE buffer. Add an equal volume of phenol/chloroform 1X, and an equal volume of chloroform 1X, and precipitate by adding 2.5 volumes 100% ethanol and 0.1 volume of 3 M NaAcetate and incubate overnight at -20°C. Pellet the DNA by centrifugation, wash with 70% ethanol, and lyophilize. Ligate double stranded cDNA to EcoRI adapters as follows:
the nucleotide sequence of the upper strand of the adapter is

```
5' CTCGAGAGAACGTATG 3';
```

the nucleotide sequence of the lower strand of the adapter is

```
5' AATTCATACGTTCTCTCGAG 3'.
```

Before ligating the adapters to the cDNA the upper strand adapter is kinased according to protocols described in Maniatis et al.

Purification of double stranded cDNA from free linkers is as follows: fractionate the entire ligation reaction through a 6% polyacrylamide gel and isolate a gel fragment containing DNA larger than 500 bp. Then electroelute the DNA from gel slice, phenol/chloroform, ethanol precipitate, centrifuge and lyophilize as described above.

Double stranded cDNA containing EcoR I adapters is ligated to lambda GT10 arms (Stratagene, La Jolla, Ca) following the protocols provided by manufacturer.

Packaging, titering and plating of cDNA library is done according to procedures supplied by the manufacturer of the lambda GT10 arms (Stratagene).

## Isolation of AHT107 Heavy Chain cDNA Clones Using Synthetic Oligonucleotide Probes

Clones representing the AHT107 heavy chain were isolated as follows: The AHT107 cDNA library was screened with three synthetic oligonucleotide probes made complementary to published mouse IgG-1 constant region nucleotide sequences. The nucleotide sequence of the probes follows:

```
probe #1:          5' GGTCACCATGGAGTTAG 3'
probe #2:          5' AGGAGCAGTTCAACAGCACT 3'
probe #3:          5' CCTGCATGATAACAGACTTC 3'
```

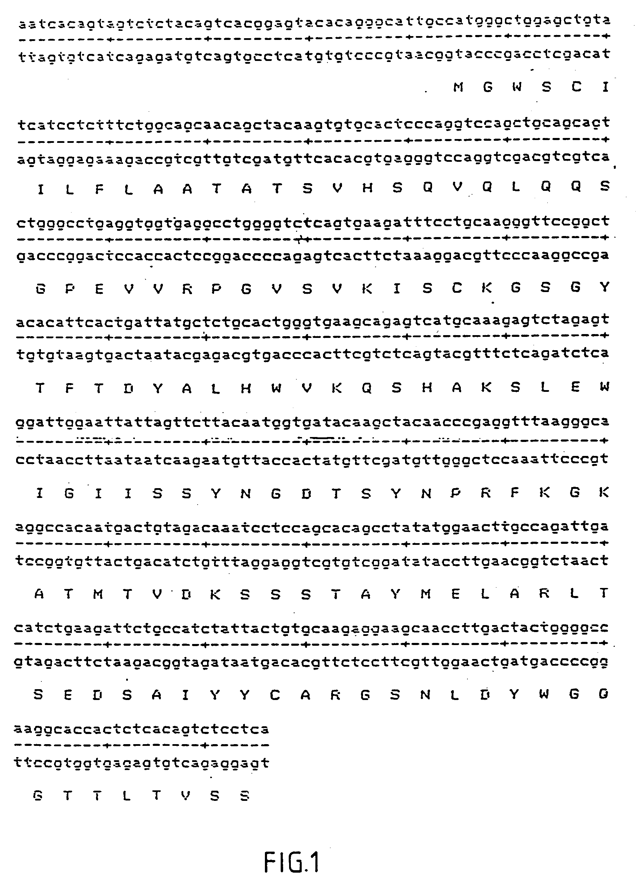

Clones that hybridized to the probes at high stringency were analyzed further by EcoR I restriction endonuclease digestion. Those clones having the largest inserts were plaque purified and their inserts subcloned into bacteriophage M13 for dideoxy sequencing. Computer-assisted analysis was used to derive the amino acid sequence of the variable region from the nucleotide sequence of the clones. The N-terminal amino acid sequence of purified AHT107 heavy chain protein was determined. Clones predicted by computer analysis to encode a full length AHT107 heavy chain protein having the same N-terminal amino acid sequence as the purified antibody were characterized further by southern analysis. Figure 1 shows the nucleotide sequence and amino acid sequence of the variable region of the AHT107 heavy chain.

### Isolation of AHT107 Heavy Chain cDNA Clones by Polymerase Chain Reaction (PCR)

The nucleotide sequence of the degenerate oligomer for priming at the 5′ end is:

ACGTTGCGAATTCTGGCCAGGTCCAGCTGCAG

$$\text{A}\quad\text{T}\quad\text{A}\quad\text{C}\quad\text{A}$$
$$\text{A}\quad\quad\text{T}\quad\text{A}$$
$$\text{G}\quad\quad\text{T}\quad\text{T}$$

The highlighted (bold) nucleotides correspond to the first five amino acids of the processed heavy chain. The overlined nucleotides correspond to EcoR I and Bal I restriction sites used for subcloning into appropriate vectors for sequencing and constructing the chimera. The nucleotide sequence of the oligomer for priming at the 3′ end is:

TACTAATGCGGAAGCTTGGGTGTCGTTTTGGC

The highlighted nucleotides correspond to the first five amino acids of the mouse IgG-1 constant region. The overlined nucleotides correspond to the Hind III restriction site used for subcloning into appropriate vectors for sequencing and constructing the chimera.

The reaction conditions for PCR are as follows:

Preincubate 10′ at 94°C. Then process for 25 cycles consisting of 1′ at 94°C; 2′ at 55°C; 3′ at 72°C. No autoextension. Post incubate 10′ at 72°C. Add equal volume of phenol/chloroform 1X, and an equal volume of chloroform 1X. Precipitate with 2.5 X volume 100% ethanol. Fractionate over a 5% polyacrylamide gel. Isolate gel fragment containing approperiate size DNA fragments. Electroelute the DNA from the gel slice. Add equal volume of phenol/chloroform 1X, and an equal volume of chloroform 1X. Precipitate with 2.5 X 100% ethanol. Resuspend in restriction endonuclease buffer and incubate with EcoR I and Hind III. Add phenol/chloroform and ethanol as above. Ligate into EcoR I/Hind III cut M13. Isolate a number of different clear plaques, determine nucleotide sequence and analyze the same as for the procedure using synthetic oligonucleotides described above.

### Construction of the AHT107 Heavy Chain Chimeric Antibody

The starting vector is pSV2NEO with the following modifications: The pSV2NEO EcoR I-BamH I fragment (bp 2635-3386) was replaced with an EcoR I-Bgl II fragment isolated from M13mp18 (bp 6231-6935). This vector is referred to as pMTI-6, a 6.5 kb Hind III-BamH I fragment containing the human IgG-1 constant region gene was subcloned as a blunt end fragment into a filled in Sal I site in the M13 polylinker of pMTI-6. This vector is referred to as pMTI-10.

The AHT107 heavy chain chimeric gene was constructed with the following components:

A Xho I-Ban I fragment from the AHT107 heavy chain cDNA clone containing all the variable region coding sequences except for the final 8 amino acids of the J region. The Xho I site of this fragment was filled in with Klenow. An approximately 8 kb BamH I-Sac I fragment isolated from pMTI-10 (the BamH I site is in the M13 Polylinker, the Sac II site is located in the CH2 domain of the human IgG-1 constant region). The BamH I site of this fragment was filled in with Klenow. An Apa I-Sac II fragment isolated from pMTI-10 (the Apa I and Sac II sites are located in the CH1 and CH2 domains of the human IgG-1 constant region, respectively). Two overlapping synthetic oligonucleotides having a Ban I site at the 5′ end and an Apa I site at the 3′ end and containing the coding sequences for the final 8 amino acids of the J region and the first few amino acids of the human constant region up to the Apa I site in the CH1 domain. The nucleotide sequence of the oligomers are:

**5'**                                                      **3'**

GCACCACTCTCACAGTCTCCTCAGCCTCCACCAAGGGCC

GTGAGAGTGTCAGAGGAGTCGGAGGTGGTTC

**3'**                                                 **5'**

The synthetic oligonucleotides were first annealed to each other and then all the components were added to a ligation reaction. Clones containing all the components were isolated by hybridization to radioactive probes. Positive clones were further characterized by southern analysis and dideoxy sequencing. One clone containing all the components in the correct configuration was chosen for further studies and it is referred to as pMTI-3101.

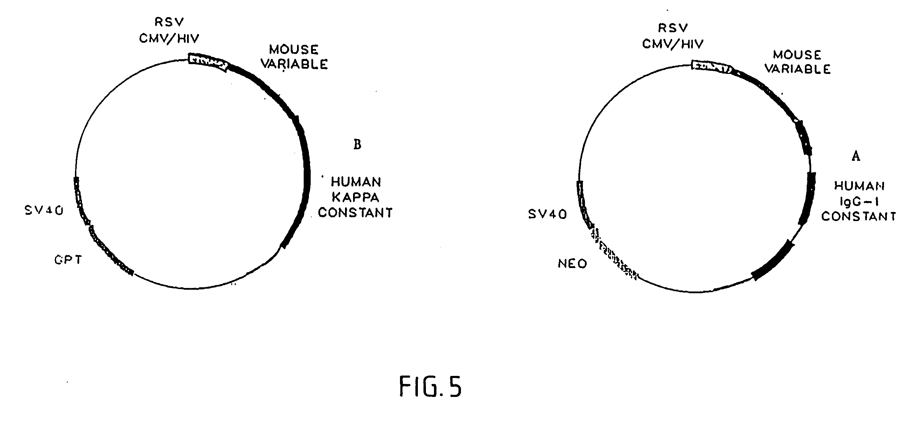

In order to express pMTI-3101 in mammalian cells various promoter and enhancer combinations were added to the vector. A 1.8 kb EcoR I-BamH I restriction fragment containing the metallothionein promoter was subcloned into the EcoR I-BamH I sites in the M13 polylinker of pMTI-3101. The resulting vector is referred to as pMTI-3103. A 600 bp Nde I - Hind III restriction fragment containing the Rous Sarcoma Virus (RSV) promoter was filled in with Klenow to produce blunt ends and subcloned into the Sma I site in the M13 polylinker of pMTI-3101. The resulting vector is referred to as pMTI-3105. A 850 bp Mlu I-Hind III restriction fragment containing the cytomegalovirus immediate early enhancer and the HIV LTR was filled in with Klenow to produce blunt ends and subcloned into the SMA I site in the M13 polylinker of pMTI-3101. The resulting vector is referred to as pMTI-3106. Figure 5A shows an expression vector for the heavy chain immunoglobulin gene.

## Expression of the Chimeric AHT107 Heavy Chain Gene

The level of expression of the three chimeric AHT107 heavy chain vectors, pMTI-3103, pMTI-3105, and pMTI-3106, were analyzed in a transient expression assay in COS cells. Transfection protocol for transient expression assay is as follows: To a sterile eppendorf tube add 10 ug DNA, 12 ul DEAE-dextran (25 mg/ml) and bring volume up to 0.6 ml with TS buffer. Remove media from a 10 cm petri dish containing $10^7$ COS cells and add DNA mix dropwise onto the cells. Incubate 60 min at 37°C in a $CO_2$ incubator. Wash with 8-10 ml TS buffer. Add 10 ml medium (DMEM + Penicillin + Streptomycin + 5% serum) containing 0.1 mM chloroquine. Incubate 3.5 h at 37°C in a $CO_2$ incubator. Remove chloroquine containing media and add fresh medium. Incubate at 37°C for 48 h. Remove media and add 250 ul RIPA lysis buffer. Incubate 20 min on ice and collect cells. Spin 5 min in eppendorf at 4°C. Remove supernatant and determine expression levels by Western and ELISA analysis. The amount of chimeric heavy chain made by pMTI-3105 is slightly higher than pMTI-3106 which is about 5 fold higher than the amount made by pMTI-3101 (data not shown).

## Isolation of AHT54 Heavy Chain cDNA Clones by Polymerase Chain Reaction (PCR)

The nucleotide sequence of the degenerate oligomer for priming at the 5' end is:

5' ACGTTGCGAATTCTCGCG**AAGTGCAGCTGGTG** 3'
                                        **G  T  A  C  T**
                                          **C     A  C**
                                          **A     G  A**
                                              **T  A**
                                              **T  G**

The highlighted (bold) nucleotides correspond to the first five amino acids of the processed heavy chain. The overlined nucleotides correspond to EcoR I and Nru I restriction sites used for subcloning into appropriate vectors for sequencing and constructing the chimeric gene. The nucleotide sequence of the oligomer for priming at the 3' end is:

5′ TACTAATGCGGAAGCTTGGGTGTCGTTTTGGC 3′

The highlighted nucleotides correspond to the first five amino acids of the mouse IgG-1 constant region. The overlined nucleotides corresponde to the Hind III restriction site used for subcloning into appropriate vectors for sequencing and constructing the chimera.

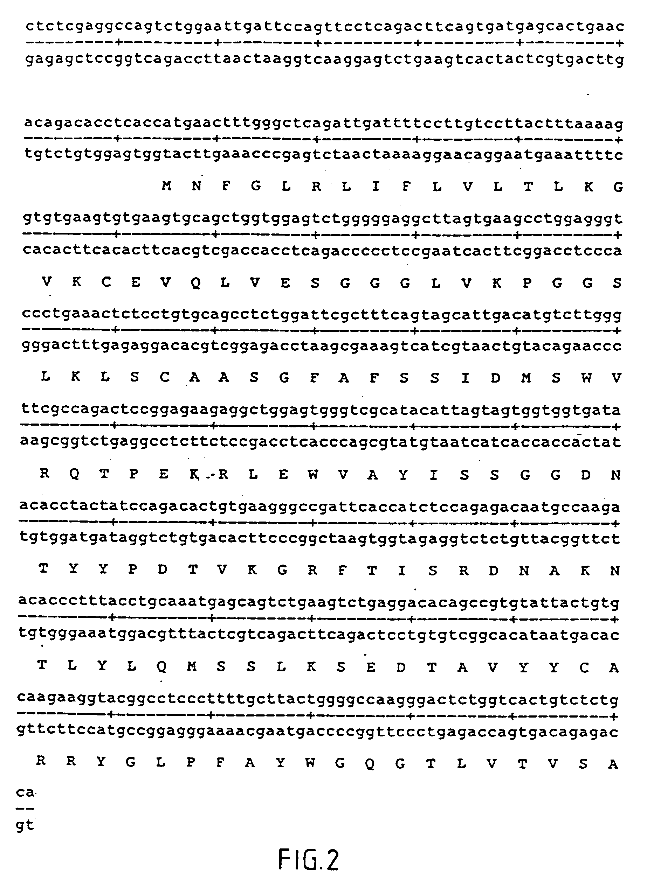

The reaction conditions for PCR are as follows: Preincubate 10′ at 94°C. Then process for 25 cycles consisting of 1′ at 94°C; 2′ at 55°C; 3′ at 72°C. No autoextension. Post incubate 10′ at 72°C. Add equal volume of phenol/chloroform 1X, and an equal volume of chloroform 1X. Precipitate with 2.5 X volume 100% ethanol. Fractionate over a 5% polyacrylamide gel. Isolate gel fragment containing appropriate size DNA fragments. Electroelute the DNA from the gel slice. Add equal volume of phenol/chloroform 1X, and an equal volume of chloroform 1X. Precipitate with 2.5 X 100% ethanol. Resuspend in restriction endonuclease buffer and incubate with EcoR I and Hind III. Add phenol/chloroform as above. Ligate into EcoR I/Hind III cut M13. Isolate a number of different clear plaques, determine nucleotide sequence and analyze the same as for the procedure using synthetic oligonucleotides described above. The nucleotide sequence and the amino acid sequence of AHT 54 heavy chain is shown in Figure 2.

## Construction of the AHT54 Heavy Chain Chimeric

The following procedure can be used for construction of the AHT54 heavy chain chimeric:

The starting vector is pSV2NEO with the following modifications: The pSV2NEO EcoR I-BamH I fragment (bp 2635 - 3386) was replaced with an EcoR I-Bgl II fragment isolated from M13mp18 (bp 6231 -6935). This vector is referred to as pMTI-6, a 6.5 kb Hind III-BamH I fragment containing the human IgG-1 constant region gene was sublconed as a blunt end fragment into a filled in Sal I site in the M13 polylinker of pMTI-6. This vector is referred to as pMTI-10.

The AHT54 heavy chain chimeric gene was constructed with the following components:

An EcoR I-Sty I fragment from the AHT54 heavy chain cDNA clone containing all the variable region coding sequences except for the final 9 amino acids of the J region. An approximately 8 kb Eco RI-Sac I fragment isolated from pMTI-10 (the Eco RI site is in the M13 polylinker, the Sac II site is located in the CH2 domain of the human IgG-1 constant region). A 1014 bp Apa I-Sac II fragment isolated from pMTI-10 (the Apa I and Sac II sites are located in the CH1 and CH2 domains of the human IgG-1 constant region, respectively). Two overlapping synthetic oligonucleotides having a Sty I site at the 5′ end and an Apa I site at the 3′ end and containing the coding sequences for the final 9 amino acids of the J region and the first few amino acids of the human constant region up to the Apa I site in the CH1 domain. The nucleotide sequence of the oligomers are:

5′ CAAGGGACTCTGGTCACTGTCTCTGCAGCCTCCACCAAGGGCC 3′

CCTGAGACCAGTGACAGAGACGTCGGAGGTGGTTC

3′                                                 5′

The synthetic oligonucleotides were first annealed to each other and then all the components were added to a ligation reaction. Clones containing all the components were isolated by hybridization to radioactive probes. Positive clones were further characterized by southern analysis and dideoxy sequencing.

A similar procedure can be utilized for the analysis and dideoxy sequencing.

A similar procedure can be utilized for the light cDNA chain and is described as follows:

## Isolation of AHT107 Light cDNA Clones Using Synthetic Oligonucleotide Probes

Clones representing the AHT107 light chain were isolated as follows: The AHT107 cDNA library was screened with a synthetic oligonucleotide probe made complementary to a published mouse kappa constant region nucleotide sequence. The nucleotide sequence of the probe is

5′ CGCCATTTTGTCGTTCACTG 3′

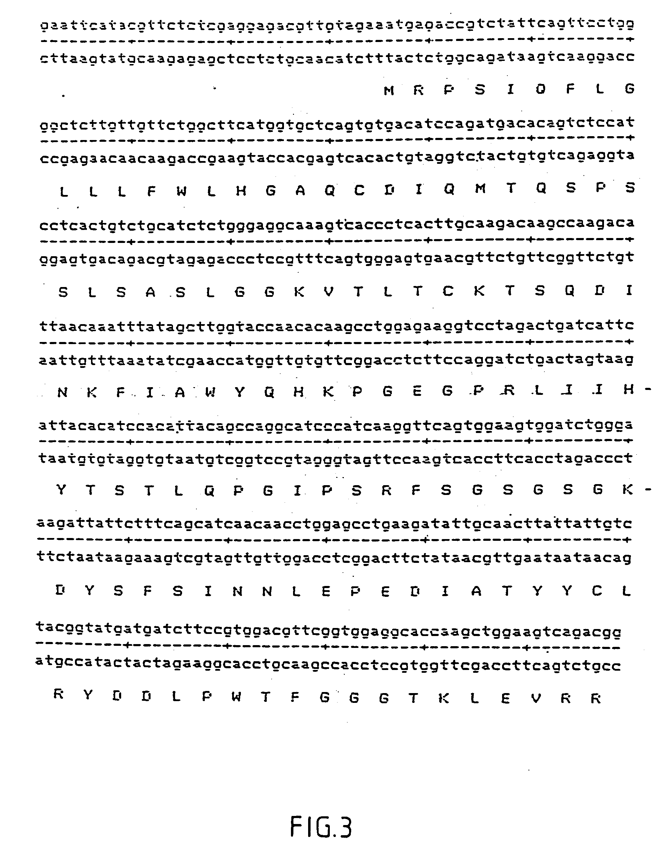

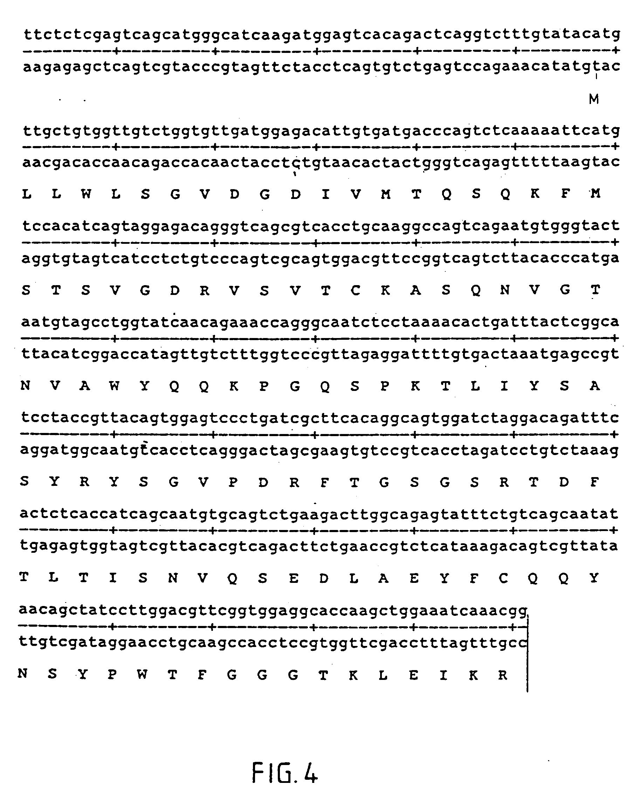

Clones that hybridized to the probe at high stringency were analyzed further by EcoR I restriction endonuclease digestion. Those clones having the largest inserts were plaque purified and their inserts subcloned into bacteriophage M13 for dideoxy sequencing. Computer-assisted analysis was used to derive the amino acid sequence of the variable region from the nucleotide sequence of the clones. The N-terminal amino acid sequence of purified AHT107 light chain protein was determined. Clones predicted by computer analysis to encode a full length AHT107 light chain protein having the same N-terminal amino acid sequence as the purified antibody were characterized further by southern analysis. Figure 3 shows the nucleotide sequence and amino acid sequence of the variable region of the AHT107 light chain. The AHT54 light chain cDNA clone was isolated and characterized as described above for the AHT107 light chain cDNA. Figure 4 shows the nucleotide sequence and amino acid sequence of the variable region of the AHT54 light chain.

**Isolation of AHT107 Light Chain cDNA Clones by Polymerase Chain Reaction (PCR)**

The AHT107 light chain can be isolated according to the following procedure.
The nucleotide sequence of the degenerate oligomer for priming at the 5′ end is:

$$5'\ \ \underline{\text{ACGTTGCGAATTCTCGCG}}\textbf{ACATCCAGATGACA}\ \ 3'$$

$$\begin{array}{cccc} \textbf{T} & \textbf{T} & \textbf{A} & \textbf{T} \\ & & \textbf{A} & \textbf{C} \\ & & & \textbf{G} \end{array}$$

The highlighted (bold) nucleotides correspond to the first five amino acids of the processed light chain. The overlined nucleotides correspond to EcoR I and Nru I restriction sites used for subcloning into appropriate vectors for sequencing and constructing the chimeric. The nucleotide sequence of the oligomer for priming at the 3′ end is:

$$5'\ \ \underline{\text{TACTAATGCGGA}}\text{AGCTT}\textbf{GCTGATGCTGCACCA}\ \ 3'$$

The highlighted nucleotides correspond to the first five amino acids of the mouse kappa constant region. The overlined nucleotides correspond to the Hind III restriction site used for subcloning into appropriate vectors for sequencing and constructing the chimera.

The reaction conditions for PCR are as follows:

Preincubate 10′ at 94°C. Then process for 25 cycles consisting of 1′ at 94°C; 2′ at 55°C; 3′ at 72°C. No autoextension. Post incubate 10′ at 72°C. Add an equal volume of phenol/chloroform 1X, and an equal volume of chloroform 1X. Precipitate with 2.5 X volume 100% ethanol. Fractionate over a 5% polyacrylamide gel. Isolate gel fragment containing appropriate size DNA fragments. Electroelute the DNA from the gel slice. Add an equal volume of phenol/chloroform 1X, and an equal volume of chloroform 1X. Precipitate with 2.5 X 100% ethanol. Resuspend in restriction endonuclease buffer and incubate with EcoR I and Hind III. Add phenol/chloroform and ethanol as above. Ligate into EcoR I/Hind III cut M13. Isolate a number of different clear plaques, determine nucleotide sequence and analyze the same as for the procedure using synthetic oligonucleotides described above. The nucleotide sequence and the amino acid sequence for the AHT107 light chain is shown in Figure 3.

**Construction of the AHT107 Light Chain Chimeric**

The AHT107 light chain chimeric was constructed as follows:

The starting vector is pSV2GPT with the following modifications: The pSV2GTP EcoR I-BamH I fragment (bp 2635 - 3386) was replaced with an EcoR I-Bgl II fragment isolated from M13mp18 (bp 6231-6935). This vector is referred to as pMTI-3050, a 2.8 kb Eco RI fragment containing the human kappa constant region gene was subcloned as a blunt end fragment into a filled in Sal I site in the M13 polylinker of pMTI-3050. This vector is referred to as pMTI-3052.

The AHT107 light chain chimeric gene was constructed with the following components:

An Xho I-Acc I fragment from the AHT107 light chain cDNA clone containing all the variable region coding sequences except for the final 20 amino acids. The Xho I site of this fragment was filled in with Klenow. An approximately 8 kb BamH I-Cla I fragment isolated from pMTI-3052. The BamH I site is in the M13 Polylinker, the Cla I site was introduced by site directed mutagenesis 37 bp downstream from the start of the human kappa constant region coding sequence. The BamH I site of this fragment was filled in with Klenow. Two overlapping synthetic oligonucleotides having a Acc I site at the 5′ end, nucleotides complementary to the Cla I overhang at the 3′ end, and containing the coding sequences for the final 20 amino acids of the variable region and the first 13 amino acids of the human kappa constant region up to the Cla I site in pMTI-3052. The nucleotide sequence of the oligomers are:

```
5′CTACGGTATGATGATCTTCCGTGGACGTTCGGTGGAGGCACCAAGCTGGA
-

3′TGCCATACTACTAGAAGGCACCTGCAAGCCACCTCCGTGGTTCGACCT-
```

```
AGTCAGACGGACTGTGGCTGCACCATCTGTCTTCATCTTCCCGCCATC 3′
TCAGTCTGCCTGACACCGACGTGGTAGACAGAAGTAGAAGGGCGGTAGGC 5′
```

The synthetic oligonucleotides were first annealed to each other and then all the components were added to a ligation reaction. Clones containing all the components were isolated by hybridization to radioactive probes. Positive clones were further characterized by southern analysis and dideoxy sequencing. One clone containing all the components in the correct configuration was chosen for further studies and it is referred to as pMTI-3056.

In order to express pMTI-3056 in mammalian cells various promoter and enhancer combinations were added to the vector. A 600 bp Nde I-Hind III restriction fragment containing the Rous Sarcoma Virus (RSV) promoter was filled in with Klenow to produce blunt ends and subcloned into the Sma I site in the M13 polylinker of pMTI-3056. The resulting vector is referred to as pMTI-3055. A 850 bp Mlu I-Hind III restriction fragment containing the Cytomegalovirus immediate early enhancer and the HIV LTR was filled in with Klenow to produce blunt ends and subcloned into the SMA I site in the M13 polylinker of pMTI-3056. The resulting vector is referred to as pMTI-3057. Figure 5B shows an expression vector for the light chain chimeric.

## Expression of the Chimeric AHT107 Light Chain Gene

The two chimeric AHT107 light chain vectors, pMTI-3055 and pMTI-3057, were initially analyzed in a transient expression assay in COS cells. (data not shown).

## Isolation of Stable Cell Lines Producing Chimeric AHT107 Antibodies

After the expression of the AHT heavy and light chain vectors, constructs were confirmed in a transient expression assay, the vectors were then used for isolation of stable cell lines. The following procedure is utilized for both heavy and light chains. Vectors (10 ug each) were linearized by restriction digestion at a non-essential part of the plasmid. The linearized vector DNAs were combined and added to AHT107 cells in a volume of 0.5 ml phosphate-buffered saline. Non-linearized vectors were also used at a concentration of 10 ug DNA/AHT107 cells/0.5 ml. The mixture was transferred to a Gene Pulser Cuvette (Bio-Rad Laboratories, Richmond, CA), placed on ice for 10 min and electroshocked. Following incubation for 10 min on ice, the cells were assayed for viability and resuspended in medium and plated at a concentration of $5 \times 10^4$ cells/ml. After incubation for 48 h, fresh medium supplemented with the appropriate drug was added. AFter 3 to 4 weeks the supernatants from drug resistant colonies were screened for the presence of chimeric light and heavy chains by ELISA. Supernatants of cultures that were positive for the presence of both antibody chains were then assayed for the ability to specifically bind to PMA stimulated human T-lymphocytes. Positive cultures were then cloned by limited dilution.

## Chimeric Monoclonal Antibodies Against TNF

TNF chimeric antibodies can be made using the same procedures and vectors that were used to construct the IL-2 receptor chimeric antibodies. Messenger RNA isolation, cDNA synthesis, construction of a Lambda GT10 library and isolation of TNF heavy and light chain cDNA clones using synthetic oligonucleotide probes are all as described above. After determining the nucleotide sequence of the light and heavy chain anti-TNF antibody variable regions, chimerics are made using overlapping synthetic oligonucleotide linkers extending from a convenient restriction site in the variable region to the unique restriction sites in the human constant regions of the previously described expression vectors. To isolate anti-TNF antibody variable region cDNAs by polymerase chain reaction, first determine the N-terminal amino acid sequence of the light and heavy chains of the antibodies. Then synthesize degenerate oligomers correspnding to the first few amino acids of the processed protein. The oligomers shold have appropriate restriction sites for subcloning and chimeric construction. The oligomers for priming at the 3′ end and the PCR protocol are as described above for the AHT107 antibodies.

## Buffers

| | |
|---|---|
| LST | 0.02 M Tris pH 7.5 |
| | 0.01 M NaCl |
| | 3 mM $MgCl_2$ |
| 4X TNLB | 5% Sucrose |
| | 1.2% Triton X-100 |
| ACE | 0.05 M NaCl |
| | 0.01 M NaAcetate pH 5.1 |
| | 3 mM EDTA |
| ACE/Phenol | 30 ml ACE buffer added to 100 ml phenol |
| TBE | 0.089 M Tris-borate |
| | 0.089 M boric acid |
| | 0.008 M EDTA |

TS Buffer
Solution 1 (2 liters):

16.0 grams NaCl
0.76 grams KCl
0.20 grams $Na_2PO_4$
6.0 grams Trizma base

Adjust pH to 7.4-7.5 with HCl. Distribute 95 ml into bottles.

Solution 2 (200 ml):

0.4 grams $MgCl_2$
0.4 grams $CaCl_2$

Autoclave solutions 1 and 2 for 20 min. Let cool. Add 5 ml solution 2 to each 95 ml bottle of solution 1. Store at room temperature.

| | |
|---|---|
| RIPA lysis buffer | 1.0% Noniodet 40 |
| | 50 mM Tris pH 8.0 |

### Increased Expression of Chimaeric Antibodies against IL-2R

The expression levels of chimaeric antibodies against IL-2R in CHO cell lines can be greatly increased, i.e. 10 fold by transfection of the HIV TAT gene into existing cell lines containing vector(s) for the heavy and light chain chimaeric antibodies. It is recognized that the HIV TAT gene in combination with the metallothionein promoter such that the TAT gene is under transcriptional regulation of the promoter, can be introduced into an existing cell line or can be incorporated into at least one vector containing the encoding sequence for the chimaeric heavy or light chain. The advantage of generating a separate vector containing the HIV TAT gene under transcriptional regulation of the metallothionein promoter, is that the vector can be used as a "reagent" vector for increasing expression levels in cell lines producing the anti-chimaeric antibodies other than IL-2R.

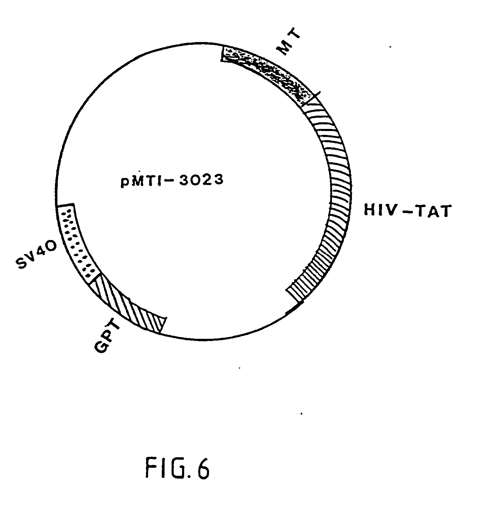

Using the expression vector of Figure 6, the expression of chimaeric antibodies can be greatly increased, i.e. 10 fold in stable transfectants that were isolated after transfection of the HIV TAT gene. The HIV TAT gene, a trans-acting factor that activates the expression of HIV genes, was localized by Arya et al, 1985. Trans-activator Gene of Human T-Lymphotropic Virus III (HILV-III), Science 229:69-73.

The expression vector, pMTI-3023 was constructed to contain the TAT gene under transcriptional regulation of the metallothionein promoter, and the GPT drug resistance marker under transcriptional regulation of the SV40 promoter, see figure 6. It is recognized that other drug resistance markers may be utilized. The vector was transfected by electroporation into the original AHT107 chimaeric antibody producing cell lines, described supra, and mycophenolic acid resistant cell lines were isolated. Experimental results demonstrated that translated TAT protein would transactivate the HIV promoter responsible for chimaeric antibody transcription. Cell lines found to be producing the highest amounts of chimaeric antibodies were cloned. A stable cell line producing the highest levels of AHT107 chimaeric antiboides was deposited as ATCC CRL10298.

## Claims

### Claims for the following Contracting States : AT, BE, CH, DE, DK, FR, GB, IT, LU, NL, SE

1. An expression vector for transfecting a host cell for producing chimeric monoclonal antibodies comprising:
   a constant region selected from the group consisting of human immunoglobin kappa light chain, human IgGI heavy chain and a combination of human immunoglobin kappa light chain and human IgGg heavy chain;
   said expression vector is adaptable, without mutagenesis, for insertion of a nonhuman antibody variable region such that the resulting expression vector includes said entire human constant region, the entire nonhuman variable region, a promotor region selected from regulatory elements of the group consisting of RSV and HIV, and an enhancer region selected from regulatory elements of the group consisting of RSV and CMV.

2. The expression vector of claim 1 **wherein said** promoter **is** upstream of said human constant region, said promoter linked to said constant region by a polylinker containing at least one restriction site, unique to said vector and to said variable region.

3. The expression vector of any of claims 1 and 2 wherein said variable region to be inserted includes a synthetic oligonucleotide linker such that when said variable region and said linker are inserted between the promoter and constant region the resulting vector includes the entire constant region and the entire nonhuman variable region fused in-frame.

4. The expression vector of claim 3 wherein said synthetic oligonucleotide linker is fused to said variable region.

5. The expression vector of any of claims 3 and 4 wherein said synthetic oligonucleotide is linked to the polymerase chain reaction primers and becomes incorporated into the amplified variable region.

6. The expression vector of any of claims 2 to 5 wherein said restriction site includes the m13MP7 polylinker.

7. The expression vector of any of claims 1-6 wherein said human constant region includes an introduced restriction site which is unique to said variable region.

8. The expression vector of claim 7 wherein said restriction site is Cla I.

9. The expression vector of any of claims 1-8 wherein the magnitude of expression of the chimeric monoclonal antibody is increased by HIV/TAT protein.

**10.** The expression vector of claims 1-9 transfected into a host cell line.

**11.** The expression vector of claim wherehin said host cell is selected from the group consisting of lymphocyte and nonlymphocyte cell lines.

**12.** A method of producing chimeric monoclonal antibodies in lymphocyte and nonlymphocyte cell lines comprising,
    (a) preparing the expression vector of claim l,
    (b) transforming an immortalized mammalian cell line with said vector of step (a), and
    (c) culturing said transformed cells to produce chimeric antibodies.

**13.** Hybridoma cell capable of producing the chimeric monoclonal antibody AHT107, said cell being ATCC CRL10030.

**14.** Hybridoma cell capable of producing the chimeric monoclonal antibody AHT107, said cell being ATCC CRL10298.

**15.** Host cell lines transformed with vectors of claims 1-9.

**Claims for the following Contracting States : ES, GR**

**1.** Method for preparing an expression vector for transfecting a host cell for producing chimeric monoclonal antibodies comprising:
    a constant region selected from the group consisting of human imunoglobin kappa light chain, human IgGl heavy chain and a combination of human immunoglobin kappa light chain and human IgGl heavy chain;
    said expression vector is adaptable, without mutagenesis, for insertion of a nonhuman antibody variable region such that the resulting expression vector includes said entire human constant region, the entire nonhuman variable region, a promotor region selected from regulatory elements of the group consisting of RSV and HIV, and an enhancer region selected from regulatory elements of the group consisting of RSV and CMV.

**2.** Method for preparing the expression vector of claim 1 **wherein said** promoter **is** upstream of said human constant region, said promoter linked to said constant region by a polylinker containing at least one restriction site, unique to said vector and to said variable region.

**3.** Method for preparing the expression vector of any of claims 1 and 2 wherein said variable region to be inserted includes a synthetic oligonucleotide linker such that when said variable region and said linker are inserted between the promoter and constant region the resulting vector includes the entire constant region and the entire nonhuman variable region fused in-frame.

**4.** Method for preparing the expression vector of claim 3 wherein said synthetic oligonucleotide linker is fused to said variable region.

**5.** Method for preparing the expression vector of any of claims 3 and 4 wherein said synthetic oligonucleotide is linked to the polymerase chain reaction primers and becomes incorporated into the amplified variable region.

**6.** Method for preparing the expression vector of any of claims 2 to 5 wherein said restriction site includes die m13MP7 polylinker.

**7.** Method for preparing the expression vector of any of claims 1-6 wherein said human constant region includes an introduced restriction site which is unique to said variable region.

**8.** Method for preparing the expression vector of claim 7 wherein said restriction site is Cla l.

**9.** Method for preparing the expression vector of any of claims 1-8 wherein the magnitude of expression of the chimeric monoclonal antibody is increased by HIV/TAT protein.

**10.** Method for preparing the expression vector of claims 1-9 transfected into a host cell line.

EP 0 380 068 B1

11. Method for preparing the expression vector of claim 10 wherehin said host cell is selected from the group consisting of lymphocyte and nonlymphocyte cell lines.

12. A method of producing chimeric monoclonal antibodies in lymphocyte and nonlymphocyte cell lines comprising,
   (a) preparing the expression vector of claim 1,
   (b) transforming an immortalized mammalian cell line with said vector of step (a), and
   (c) culturing said transformed cells to produce chimeric antibodies.

13. Method for preparing a Hybridoma cell capable of producing the chimeric monoclonal antibody AHT 107, said cell being ATCC CRL10030.

14. Method for preparing a Hybridoma cell capable of producing the chimeric monoclonal antibody AHTI07, said cell being ATCC CRL10298.

15. Method for preparing host cell lines transformed with vectors of claims 1 - 9.


**Patentansprüche**

**Patentansprüche für folgende Vertragsstaaten : AT, BE, CH, DE, DK, FR, GB, IT, LI, LU, NL, SE**

1. Expressionsvektor zur Transfektion einer Wirtszelle zur Produktion chimärer monoklonaler Antikörper umfassend:
   eine konstante Region ausgewählt aus der Gruppe bestehend aus der leichten kappa-Kette von humanem Immunglobulin, der schweren Kette von humanem IgG1 und einer Kombination der leichten kappa-Kette von humanem Immunglobulin und der schweren Kette von humanem IgG1;
   wobei der Expressionsvektor ohne Mutagenese für die Insertion einer variablen Region eines nichthumanen Antikörpers adaptierbar ist, so daß der erhaltene Expressionsvektor die gesamte humane konstante Region, die gesamte nichthumane variable Region, eine Promotorregion ausgewählt aus regulatorischen Elementen aus der Gruppe bestehend aus RSV und HIV, und eine Enhancerregion ausgewählt aus den regulatorischen Elementen aus der Gruppe bestehend aus RSV und CMV enthält.

2. Expressionsvektor nach Anspruch 1, worin der Promotor stromaufwärts der humanen konstanten Region liegt, wobei der Promotor an die konstante Region über einen Polylinker gebunden ist, der mindestens eine Schnittstelle, die einmalig für den Vektor und für die variable Region ist, enthält.

3. Expressionsvektor nach einem der Ansprüche 1 und 2, worin die einzufügende variable Region einen synthetischen Oligonukleotidlinker enthält, so daß, wenn die variable Region und der genannte Linker zwischen dem Promotor und der konstanten Region eingefügt wird, der erhaltene Vektor die gesamte konstante Region und die gesamte nichthumane variable Region im Leserahmen fusioniert enthält.

4. Expressionsvektor nach Anspruch 3, worin der synthetische Oligonukleotidlinker an die variable Region fusioniert ist.

5. Expressionsvektor nach einem der Ansprüche 3 und 4, worin das synthetische Oligonukleotid an die Primer der Polymerase-Kettenreaktion gebunden ist und in die amplifizierte variable Region eingebaut wird.

6. Expressionsvektor nach einem der Ansprüche 2 bis 5, worin die Restriktions-Schnittstelle den m13MP7-Polylinker umfaßt.

7. Expressionsvektor nach einem der Ansprüche 1 bis 6, worin die humane konstante Region eine eingeführte Restriktions-Schnittstelle, die einmalig für die variable Region ist, umfaßt.

8. Expressionsvektor nach Anspruch 7, worin die Restriktionsstelle ClaI ist.

9. Expressionsvektor nach einem der Ansprüche 1 bis 8, worin die Größenordnung der Expression der chimären monoklonalen Antikörper durch HIV/TAT Protein gesteigert wird.

10. Expressionsvektor nach den Ansprüchen 1 bis 9, transfektiert in eine Wirtszellinie.

14

**11.** Expressionsvektor nach Anspruch 10, worin die Wirtszellinie ausgewählt ist aus der Gruppe bestehend aus lymphozytischen und nichtlymphozytischen Zellinien.

**12.** Verfahren zur Herstellung chimärer monoklonaler Antikörper in lymphozytischen und nichtlymphozytischen Zellinien umfassend
   (a) Herstellen des Expressionsvektors nach Anspruch 1,
   (b) Transformation einer unsterblichen Säugerzellinie mit einem Vektor aus Schritt (a) und
   (c) Kultur der transformierten Zellen zur Produktion chimärer Antikörper.

**13.** Hybridomazelle, fähig zur Produktion des chimären monoklonalen Antikörpers AHT107, wobei die Zelle ATCC CRL10030 ist.

**14.** Hybridomazelle, fähig zur Produktion des chimären monoklonalen Antikörpers AHT107, wobei die Zelle ATCC CRL10298 ist.

**15.** Wirtszellinien, transformiert mit Vektoren der Ansprüche 1 bis 9.

**Patentansprüche für folgende Vertragsstaaten : ES, GR**

**1.** Verfahren zur Herstellung eines Expressionsvektors zur Transfektion einer Wirtszelle zur Produktion chimärer monoklonaler Antikörper umfassend:
   eine konstante Region ausgewählt aus der Gruppe bestehend aus der leichten kappa-Kette von humanem Immunglobulin, der schweren Kette von humanem IgG1 und einer Kombination der leichten kappa-Kette von humanem Immunglobulin und der schweren Kette von humanem IgG1;
   wobei der Expressionsvektor ohne Mutagenese für die Insertion einer variablen Region eines nichthumanen Antikörpers adaptierbar ist, so daß der erhaltene Expressionsvektor die gesamte humane konstante Region, die gesamte nichthumane variable Region, eine Promotorregion ausgewählt aus regulatorischen Elementen aus der Gruppe bestehend aus RSV und HIV, und eine Enhancerregion ausgewählt aus den regulatorischen Elementen aus der Gruppe bestehend aus RSV und CMV enthält.

**2.** Verfahren zur Herstellung des Expressionsvektors nach Anspruch 1, worin der Promotor stromaufwärts der humanen konstanten Region liegt, wobei der Promotor an die konstante Region über einen Polylinker gebunden ist, der mindestens eine für den Vektor und die variable Region einmalige Schnittstelle enthält.

**3.** Verfahren zur Herstellung des Expressionsvektors nach einem der Ansprüche 1 und 2, worin die einzufügende variable Region einen synthetischen Oligonukleotidlinker enthält, so daß, wenn die variable Region und der Linker zwischen dem Promotor und der konstanten Region eingefügt -erden, der erhaltene Vektor die gesamte konstante Region und die gesamte nichthumane variable Region im Leserahmen fusioniert enthält.

**4.** Verfahren zur Herstellung des Expressionsvektors nach Anspruch 3, worin der synthetische Oligonukleotidlinker an die variable Region fusioniert ist.

**5.** Verfahren zur Herstellung des Expressionsvektors nach einem der Ansprüche 3 und 4, worin das synthetische Oligonukleotid an die Primer der Polymerase-Kettenreaktion gebunden ist und in die amplifizierte variable Region eingebaut wird.

**6.** Verfahren zur Herstellung des Expressionsvektors nach einem der Ansprüche 2 bis 5, worin die Restriktions-Schnittstelle den m13MP7--Polylinker umfaßt.

**7.** Verfahren zur Herstellung des Expressionsvektors nach einem der Ansprüche 1 bis 6, worin die humane konstante Region eine eingefügte Restriktions-Schnittstelle enthält, die für die variable Region einmalig ist.

**8.** Verfahren zur Herstellung des Expressionsvektors nach Anspruch 7, worin die Restriktions-Schnittstelle ClaI ist.

**9.** Verfahren zur Herstellung des Expressionsvektors nach einem der Ansprüche 1 bis 8, worin die Größenordnung der Expression des chimären monoklonalen Antikörpers durch HIV/TAT Protein gesteigert wird.

**10.** Verfahren zur Herstellung des Expressionsvektors der Ansprüche 1 bis 9, transfektiert in eine Wirtszellinie.

**11.** Verfahren zur Herstellung des Expressionsvektors aus Ansprüch 10, worin die Wirtszelle ausgewählt ist aus der Gruppe bestehend aus lymphozytischen und nichtlymphozytischen Zellinien.

**12.** Verfahren zur Herstellung chimärer monoklonaler Antikörper in lymphozytischen und nichtlymphozytischen Zellinien umfassend
(a) Herstellen des Expressionsvektors aus Anspruch 1,
(b) Transformation einer unsterblichen Säugerzellinie mit dem Vektor aus Schritt (a) und
(c) Kultur der transformierten Zellen zur Produktion chimärer Antikörper.

**13.** Verfahren zur Herstellung einer Hybridomazelle, die zur Produktion des chimären monoklonalen Antikörpers AHT107 in der Lage ist, wobei die Zelle ATCC CRL10030 ist.

**14.** Verfahren zur Herstellung einer Hybridomazelle, die zur Produktion des chimären monoklonalen Antikörpers AHT107 in der Lage ist, wobei die Zelle ATCC CRL10298 ist.

**15.** Verfahren zur Herstellung von Wirtszellinien, transformiert mit Vektoren der Ansprüche 1 bis 9.

## Revendications

### Revendications pour les Etats contractants suivants : AT, BE, CH, DE, DK, FR, GB, IT, LI, LU, NL, SE

**1.** Un vecteur d'expression pour la transfection d'une cellule hâte pour la production d'anticorps monoclonaux chimériques comprenant:
une région constante choisie dans le groupe composé de la chaîne légère de l'immunoglobuline humaine kappa, de la chaîne lourde de l'IgG-1 humaine et d'une combinaison d'une chaîne légère de l'immunoglobuline humaine kappa et d'une chaîne lourde de l'IgG1 humaine;
ledit vecteur d'expression étant adaptable sans mutagenèse en vue de l'insertion d'une région variable de l'anticorps non humain de sorte que le vecteur d'expression comprenne ladite région constante humaine entière, la région variable non humaine entière, une région de promoteur choisie parmi les éléments régulateurs du groupe composé du RSV et HIV et une région d'induction choisie parmi les éléments régulateurs du groupe composé du RSV et CMV.

**2.** Le vecteur d'expression selon la revendication 1, **dans lequel ledit** promoteur **est** en amont de ladite région constante humaine, ledit promoteur lié à ladite région constante par un agent de liaisons multiples contenant au moins un site de restriction unique audit vecteur et à ladite région variable.

**3.** Le vecteur d'expression selon une des revendications 1 et 2, dans lequel ladite région variable à insérer comprend un liant d'oligonucléotides synthétiques de sorte que, lorsque ladite région variable et ledit liant sont insérés entre le promoteur et la région constante, le vecteur obtenu comprend toute la région constante et toute la région variable non humaine fusionnées in situ.

**4.** Le vecteur d'expression selon la revendication 3, dans lequel ledit liant d'oligonucléotides synthétiques est fusionné à ladite région variable.

**5.** Le vecteur d'expression selon une des revendications 3 et 4, dans lequel ledit oligonucléotide synthétique est lié aux amorces de la réaction de chaîne de la polymérase et est incorporé dans la région variable amplifiée.

**6.** Le vecteur d'expression selon une des revendications 2 à 5, dans lequel ledit site de restriction comprend l'agent de liaisons multiples m13MP7.

**7.** Le vecteur d'expression selon une des revendications 1 à 6, dans lequel ladite région constante humaine comprend un site de restriction introduit qui est unique à ladite région variable.

**8.** Le vecteur d'expression de la revendication 7, dans lequel ledit site de restriction est Cla I.

**9.** Le vecteur d'expression selon une des revendications 1 à 8, dans lequel l'importance de l'expression de l'anticorps monoclonal chimérique est augmentée par la protéine HIV/TAT.

**10.** Le vecteur d'expression selon une des revendications 1 à 9,transfecté dans une souche de cellule hôte.

**11.** Le vecteur d'expression selon la revendication 10, dans lequel ladite cellule hôte est choisie dans le groupe composé de souches de cellules lymphocytes et non lymphocytes.

**12.** Une méthode de production dans des anticorps monoclonaux chimériques dans des souches de cellules lymphocytes et non lymphocytes comprenant:
(a) la préparation du vecteur d'expression de la revendication 1,
(b) la transformation d'une souche de cellules mammifères immortalisées avec ledit vecteur de l'étape (a) et
(c) la culture desdites cellules transformées pour produire des anticorps chimériques.

**13.** La cellule hybridome capable de produire l'anticorps monoclonal chimérique AHT107, ladite cellule étant l'ATCC CRL10030.

**14.** La cellule hybridome de production d'un anticorps monoclonal chimérique AHT107, ladite cellule étant l'ATCC CRL10298.

**15.** Les souches de cellule hôte transformées avec les vecteurs des revendications 1 à 9.

**Revendications pour les Etats contractants suivants : ES, GR**

**1.** Méthode de préparation d'un vecteur d'expression pour la transfection d'une cellule hôte pour la production d'anticorps monoclonaux chimériques comprenant:
une région constante choisie dans le groupe composé de la chaîne légère de l'immunoglobuline humaine kappa, de la chaîne lourde de l'IgG-1 humaine et d'une combinaison d'une chaîne légère de l'immunoglobuline humaine kappa et d'une chaîne lourde de l'IgGl humaine;
ledit vecteur d'expression étant adaptable sans mutagenèse en vue de l'insertion d'une région variable de l'anticorps non humain de sorte que le vecteur d'expression comprenne ladite région constante humaine entière, la région variable non humaine entière, une région de promoteur choisie parmi les éléments régulateurs du groupe composé du RSV et HIV et une région d'induction choisie parmi les éléments régulateurs du groupe composé du RSV et CMV.

**2.** Méthode de préparation du vecteur d'expression selon la revendication 1, dans laquelle **ledit** promoteur **est** en amont de ladite région constante humaine, ledit promoteur lié à ladite région constante par un agent de liaisons multiples contenant au moins un site de restriction unique audit vecteur et à ladite région variable.

**3.** Méthode de préparation du vecteur d'expression selon une des revendications 1 et 2, dans laquelle ladite région variable à insérer comprend un liant d'oligonucléotides synthétiques de sorte que, lorsque ladite région variable et ledit liant sont insérés entre le promoteur et la région constante, le vecteur obtenu comprend toute la région constante et toute la région variable non humaine fusionnées in situ.

**4.** Méthode de préparation du vecteur d'expression selon la revendication 3, dans laquelle ledit liant d'oligonucléotides synthétiques est fusionné à ladite région variable.

**5.** Méthode de préparation du vecteur d'expression selon une des revendications 3 et 4, dans laquelle ledit oligonucléotide synthétique est lié aux amorces de la réaction de chaîne de la polymérase et est incorporé dans la région variable amplifiée.

**6.** Méthode de préparation du vecteur d'expression selon une des revendications 2 à 5, dans laquelle ledit site de restriction comprend l'agent de liaisons multiples m13MP7.

**7.** Méthode de préparation du vecteur d'expression selon une des revendications 1 à 6, dans laquelle ladite région constante humaine comprend un site de restriction introduit qui est unique à ladite région variable.

**8.** Méthode de préparation du vecteur d'expression de la revendication 7, dans laquelle ledit site de restriction est Cla I.

9. Méthode de préparation du vecteur d'expression selon une des revendications 1 à 8, dans laquelle l'importance de l'expression de l'anticorps monoclonal chimérique est augmentée par la protéine HIV/TAT.

10. Méthode de préparation du vecteur d'expression selon une des revendications 1 à 9 transfecté dans une souche de cellule hôte.

11. Méthode de préparation du vecteur d'expression selon la revendication 10, dans laquelle ladite cellule hôte est choisie dans le groupe composé de souches de cellules lymphocytes et non lymphocytes.

12. Une méthode de production dans des anticorps monoclonaux chimériques dans des souches de cellules lymphocytes et non lymphocytes comprenant:
    (a) la préparation du vecteur d'expression de la revendication 1,
    (b) la transformation d'une souche de cellules mammifères immortalisées avec ledit vecteur de l'étape (a) et
    (c) la culture desdites cellules transformées pour produire des anticorps chimériques.

13. Méthode de préparation d'une cellule hybridome capable de produire l'anticorps monoclonal chimérique AHT107, ladite cellule étant l'ATCC CRL10030.

14. Méthode de préparation d'une cellule hybridome de production d'un anticorps monoclonal chimérique AHT107, ladite cellule étant l'ATCC CRL10298.

15. Méthode de préparation de souches de cellule hôte transformées avec les vecteurs des revendications 1 à 9.

```
aatcacagtagtctctacagtcacggagtacacagggcattgccatgggctggagctgta
---------+---------+---------+---------+---------+---------+
ttagtgtcatcagagatgtcagtgcctcatgtgtcccgtaacggtacccgacctcgacat
                                        M   G   W   S   C   I

tcatcctctttctggcagcaacagctacaagtgtgcactcccaggtccagctgcagcagt
---------+---------+---------+---------+---------+---------+
agtaggagaaagaccgtcgttgtcgatgttcacacgtgagggtccaggtcgacgtcgtca
  I   L   F   L   A   A   T   A   T   S   V   H   S   Q   V   Q   L   Q   Q   S

ctggccctgaggtggtgaggcctggggtctcagtgaagatttcctgcaagggttccggct
---------+---------+---------+---------+---------+---------+
gaccgggactccaccactccggaccccagagtcacttctaaaggacgttcccaaggccga
  G   P   E   V   V   R   P   G   V   S   V   K   I   S   C   K   G   S   G   Y

acacattcactgattatgctctgcactgggtgaagcagagtcatgcaaagagtctagagt
---------+---------+---------+---------+---------+---------+
tgtgtaagtgactaatacgagacgtgacccacttcgtctcagtacgtttctcagatctca
  T   F   T   D   Y   A   L   H   W   V   K   Q   S   H   A   K   S   L   E   W

ggattggaattattagttcttacaatggtgatacaagctacaacccgaggtttaagggca
---------+---------+---------+---------+---------+---------+
cctaaccttaataatcaagaatgttaccactatgttcgatgttgggctccaaattcccgt
  I   G   I   I   S   S   Y   N   G   D   T   S   Y   N   P   R   F   K   G   K

aggccacaatgactgtagacaaatcctccagcacagcctatatggaacttgccagattga
---------+---------+---------+---------+---------+---------+
tccggtgttactgacatctgtttaggaggtcgtgtcggatataccttgaacggtctaact
  A   T   M   T   V   D   K   S   S   S   T   A   Y   M   E   L   A   R   L   T

catctgaagattctgccatctattactgtgcaagaggagcaaccttgactactggggcc
---------+---------+---------+---------+---------+---------+
gtagacttctaagacggtagataatgacacgttctccttcgttggaactgatgaccccgg
  S   E   D   S   A   I   Y   Y   C   A   R   G   S   N   L   D   Y   W   G   D

aaggcaccactctcacagtctcctca
---------+---------+------
ttccgtggtgagagtgtcagaggagt
  G   T   T   L   T   V   S   S
```

# FIG.1

```
ctctcgaggccagtctggaattgattccagttcctcagacttcagtgatgagcactgaac
--------+---------+---------+--------+--------+---------+
gagagctccggtcagaccttaactaaggtcaaggagtctgaagtcactactcgtgacttg


acagacacctcaccatgaactttgggctcagattgattttccttgtccttactttaaaag
--------+---------+---------+--------+--------+---------+
tgtctgtggagtggtacttgaaacccgagtctaactaaaaggaacaggaatgaaattttc

          M   N   F   G   L   R   L   I   F   L   V   L   T   L   K   G

gtgtgaagtgtgaagtgcagctggtggagtctggggggaggcttagtgaagcctggagggt
--------+---------+---------+--------+--------+---------+
cacacttcacacttcacgtcgaccacctcagacccctccgaatcacttcggacctccca

   V   K   C   E   V   Q   L   V   E   S   G   G   G   L   V   K   P   G   G   S

ccctgaaactctcctgtgcagcctctggattcgctttcagtagcattgacatgtcttggg
--------+---------+---------+--------+--------+---------+
gggactttgagaggacacgtcggagacctaagcgaaagtcatcgtaactgtacagaaccc

   L   K   L   S   C   A   A   S   G   F   A   F   S   S   I   D   M   S   W   V

ttcgccagactccggagaagaggctggagtgggtcgcatacattagtagtggtggtgata
--------+---------+---------+--------+--------+---------+
aagcggtctgaggcctcttctccgacctcacccagcgtatgtaatcatcaccaccactat

   R   Q   T   P   E   K   R   L   E   W   V   A   Y   I   S   S   G   G   D   N

acacctactatccagacactgtgaagggccgattcaccatctccagagacaatgccaaga
--------+---------+---------+--------+--------+---------+
tgtggatgataggtctgtgacacttcccggctaagtggtagaggtctctgttacggttct

   T   Y   Y   P   D   T   V   K   G   R   F   T   I   S   R   D   N   A   K   N

acaccctttacctgcaaatgagcagtctgaagtctgaggacacagccgtgtattactgtg
--------+---------+---------+--------+--------+---------+
tgtgggaaatggacgtttactcgtcagacttcagactcctgtgtcggcacataatgacac

   T   L   Y   L   Q   M   S   S   L   K   S   E   D   T   A   V   Y   Y   C   A

caagaaggtacggcctcccttttgcttactggggccaagggactctggtcactgtctctg
--------+---------+---------+--------+--------+---------+
gttcttccatgccggagggaaaacgaatgaccccggttccctgagaccagtgacagagac

   R   R   Y   G   L   P   F   A   Y   W   G   Q   G   T   L   V   T   V   S   A

ca
--
gt
```

FIG.2

EP 0 380 068 B1

```
gaattcatacgttctctcgaggagacgttgtagaaatgagaccgtctattcagttcctgg
----------+---------+---------+---------+---------+---------+
cttaagtatgcaagagagctcctctgcaacatctttactctggcagataagtcaaggacc
                                       M  R  P  S  I  Q  F  L  G

ggctcttgttgttctggcttcatggtgctcagtgtgacatccagatgacacagtctccat
----------+---------+---------+---------+---------+---------+
ccgagaacaacaagaccgaagtaccacgagtcacactgtaggtctactgtgtcagaggta
 L  L  L  F  W  L  H  G  A  Q  C  D  I  Q  M  T  Q  S  P  S

cctcactgtctgcatctctgggaggcaaagtcaccctcacttgcaagacaagccaagaca
----------+---------+---------+---------+---------+---------+
ggagtgacagacgtagagaccctccgtttcagtgggagtgaacgttctgttcggttctgt
  S  L  S  A  S  L  G  G  K  V  T  L  T  C  K  T  S  Q  D  I

ttaacaaatttatagcttggtaccaacacaagcctggagaaggtcctagactgatcattc
----------+---------+---------+---------+---------+---------+
aattgtttaaatatcgaaccatggttgtgttcggacctcttccaggatctgactagtaag
   N  K  F  I  A  W  Y  Q  H  K  P  G  E  G  P  R  L  I  I  H -

attacacatccacattacagccaggcatccatcaaggttcagtggaagtggatctggca
----------+---------+---------+---------+---------+---------+
taatgtgtaggtgtaatgtcggtccgtagggtagttccaagtcaccttcacctagaccct
  Y  T  S  T  L  Q  P  G  I  P  S  R  F  S  G  S  G  S  G  K -

aagattattctttcagcatcaacaacctggagcctgaagatattgcaacttattattgtc
----------+---------+---------+---------+---------+---------+
ttctaataagaaagtcgtagttgttggacctcggacttctataacgttgaataataacag
  D  Y  S  F  S  I  N  N  L  E  P  E  D  I  A  T  Y  Y  C  L

tacggtatgatgatcttccgtggacgttcggtggaggcaccaagctggaagtcagacgg
----------+---------+---------+---------+---------+---------
atgccatactactagaaggcacctgcaagccacctccgtggttcgaccttcagtctgcc
  R  Y  D  D  L  P  W  T  F  G  G  G  T  K  L  E  V  R  R
```

FIG.3

21

```
ttctctcgagtcagcatgggcatcaagatggagtcacagactcaggtctttgtatacatg
---------+---------+---------+---------+---------+---------+
aagagagctcagtcgtacccgtagttctacctcagtgtctgagtccagaaacatatgtac

                                                            M

ttgctgtggttgtctggtgttgatggagacattgtgatgacccagtctcaaaaattcatg
---------+---------+---------+---------+---------+---------+
aacgacaccaacagaccacaactacctctgtaacactactgggtcagagttttttaagtac

L   L   W   L   S   G   V   D   G   D   I   V   M   T   Q   S   Q   K   F   M

tccacatcagtaggagacagggtcagcgtcacctgcaaggccagtcagaatgtgggtact
---------+---------+---------+---------+---------+---------+
aggtgtagtcatcctctgtcccagtcgcagtggacgttccggtcagtcttacacccatga

S   T   S   V   G   D   R   V   S   V   T   C   K   A   S   Q   N   V   G   T

aatgtagcctggtatcaacagaaaccagggcaatctcctaaaacactgatttactcggca
---------+---------+---------+---------+---------+---------+
ttacatcggaccatagttgtctttggtcccgttagaggattttgtgactaaatgagccgt

N   V   A   W   Y   Q   Q   K   P   G   Q   S   P   K   T   L   I   Y   S   A

tcctaccgttacagtggagtccctgatcgcttcacaggcagtggatctaggacagatttc
---------+---------+---------+---------+---------+---------+
aggatggcaatgtcacctcagggactagcgaagtgtccgtcacctagatcctgtctaaag

S   Y   R   Y   S   G   V   P   D   R   F   T   G   S   G   S   R   T   D   F

actctcaccatcagcaatgtgcagtctgaagacttggcagagtatttctgtcagcaatat
---------+---------+---------+---------+---------+---------+
tgagagtggtagtcgttacacgtcagacttctgaaccgtctcataaagacagtcgttata

T   L   T   I   S   N   V   Q   S   E   D   L   A   E   Y   F   C   Q   Q   Y

aacagctatccttggacgttcggtggaggcaccaagctggaaatcaaacgg
---------+---------+---------+---------+---------+-
ttgtcgataggaacctgcaagccacctccgtggttcgacctttagtttgcc

N   S   Y   P   W   T   F   G   G   G   T   K   L   E   I   K   R
```

FIG.4

22

FIG.5

FIG. 6